(19) Europäisches Patentamt
European Patent Office
Office européen des brevets

(11) Publication number: 0 336 440 A2

# (12) EUROPEAN PATENT APPLICATION

(21) Application number: 89106198.8

(51) Int. Cl.4: C07C 29/15 , C07C 31/04

(22) Date of filing: 07.04.89

(30) Priority: 07.04.88 US 178953

(43) Date of publication of application: 11.10.89 Bulletin 89/41

(84) Designated Contracting States: BE DE ES FR GB IT NL SE

(71) Applicant: AIR PRODUCTS AND CHEMICALS, INC.
Route no. 222
Trexlertown Pennsylvania 18087(US)

(72) Inventor: Hsiung, Thomas Hsiao-Ling
4727 Glenwood Circle
Emmaus, PA 18049(US)
Inventor: Perka, Alan Thomas
1911 Aster Road
Macungie, PA 18062(US)
Inventor: Klosek, Joseph
1416 Mashie Drive
Wescosville, PA 18106(US)
Inventor: Moore, Robert Byron
2951 Edgemont Court
Allentown, PA 18103(US)

(74) Representative: Dipl.-Ing. Schwabe, Dr. Dr. Sandmair, Dr. Marx
Stuntzstrasse 16
D-8000 München 80(DE)

## (54) Process using water addition for the conversion of co-rich synthesis gas to methanol.

(57) The present invention relates to an improvement to a process for the production of methanol from synthesis gas containing carbon monoxide and hydrogen utilizing a three phase or liquid phase reaction technology. The improvement to the process is the addition of relatively large amounts of water to the liquid phase reactor thereby allowing for the use of a CO-rich synthesis gas for the production of methanol by effectuating in the same reactor the methanol synthesis and water-gas shift reactions.

EP 0 336 440 A2

# PROCESS USING WATER ADDITION FOR THE CONVERSION OF CO-RICH SYNTHESIS GAS TO METHANOL

## TECHNICAL FIELD

The present invention relates to a process for the production of methanol from a synthesis gas containing carbon monoxide and hydrogen using a three phase or liquid phase reaction technology. More specifically, the present invention relates to an improvement allowing the use of CO-rich synthesis gas in the methanol synthesis process wherein relatively large quantities of water are added to the liquid phase reactor.

## BACKGROUND OF THE INVENTION

Methanol is produced from synthesis gas (syngas), a mixture of hydrogen ($H_2$), carbon monoxide (CO), and carbon dioxide ($CO_2$). The stoichiometry of the methanol synthesis reactions indicates that the desired molar reactor feed composition is given by the equation:

$R = (H_2\text{-}CO_2)/(CO + CO_2) = 2.0$

However, reaction kinetics and system control dictate that the optimum ratio is actually R = 2.1 or higher. Gas with R = 2.0 to 2.1 is called "balanced" gas, i.e. balanced stoichimetrically, and has a typical composition of 19% CO, 5% $CO_2$, 55% $H_2$, and 21% $CH_4$-$N_2$.

Syngas is commonly made by the reforming of methane or other hydrocarbons, which gives a hydrogen-rich gas well-suited for methanol synthesis (e.g., a typical methanol syngas produced by steam reforming of methane has a composition of 15% CO, 8% $CO_2$, 73% H2, 4% $CH_4$-N2, R = 2.8). Currently 70 to 75% of the world's methanol comes from reformed natural gas, however, because of the instability of the oil market, liquid hydrocarbons and natural gas are not always readily available or available at an inexpensive cost. An alternative and abundant resource is coal, which can be converted to syngas in a coal gasifier such as the advanced, high-temperature coal gasifiers developed by Texaco, Dow, Shell and British Gas/Lurgi.

Unfortunately, coal-derived syngas from advanced gasifiers is CO-rich (e.g., a Texaco gasifier has a typical composition of 35% $H_2$, 51% CO, 13% $CO_2$, 1% $CH_4$-$N_2$; R = 0.34), unlike the hydrogen-rich syngas from reformed hydrocarbons. The problem is that converting this gas to methanol by conventional methods is expensive and complicated because several pretreatment steps are required to balance the gas prior to methanol synthesis.

The conventional plant concept is to pretreat the raw syngas and then convert it to methanol in a synthesis reactor operated with recycle. This is referred to as the "all-methanol" approach. The objective is to convert as much of the carbon input to the process as possible into methanol. The following describes the general configuration of a conventional all-methanol plant, as well as four types of methanol synthesis processes which could be used in such a plant: the conventional gas-phase reactor type process, the liquid-phase reactor type process, the ICI "dual cycle" process, and the Tarhan process.

Almost all of the world's methanol is currently made in conventional, gas-phase, all-methanol processes. These require that the syngas composition be balanced or $H_2$-rich prior to methanol synthesis. This is not only to give optimum performance and control; there is also evidence that excess CO accelerates catalyst deactivation. The reason for this is not clear. Two possible explanations are that CO poisons the catalyst at high concentrations (either directly or indirectly through carbon deposition), or that high CO concentrations cause excessive local temperature rises, "hot spots", which damage the temperature sensitive catalyst.

The CO-rich syngas is traditionally balanced in two steps, water-gas shift and $CO_2$ removal. First, water vapor is added to a portion of the gas, and the mixture is passed over a catalyst which promotes the shift reaction (CO + $H_2O$ = $CO_2$ + H2). Conventional shift reactors are of two types. The first type is an adiabatic packed bed of catalyst. Depending on the amount of CO to be converted, one or more shift beds are used. These units are typically run in two temperature ranges. High temperature shift (HTS) uses a chromium-promoted iron catalyst, and operates at 625 to 930 °F. Carbon monoxide concentrations can be reduced to about 2% using HTS. Low temperature shift (LTS) uses a CuO-ZnO-$Al_2O_3$ catalyst, operates at 380 to 475 °F, and can further reduce the CO concentration to about 0.2%. Depending on the feed gas and desired conversion of CO to $H_2$, a combination of HTS and LTS reactors may be used. When more than one bed is used, the gas is cooled by direct or indirect heat exchange between vessels.

The second type of conventional shift reactor is the Lurgi "isothermal" reactor. It is configured somewhat like a vertical shell-and-tube heat exchanger. The feed gas enters the bottom of the shell side and is preheated as it flows up past the tubes, which are packed with shift catalyst. The gas then passes down through the tubes, where the shift reaction takes place. The heat of reaction is removed by the cool feed flowing on the shell side. Although there is a temperature profile along the tubes, the temperature at tube inlet is the same as that at outlet, hence the term "isothermal". This type of reactor operates from about 650 to 800 °F, using HTS catalyst. Carbon monoxide content can be reduced to around 7.5%.

The shifting process not only increases the $H_2$ content of the syngas, but the $CO_2$ content as well. The $CO_2$ content is increased to the point where some $CO_2$ must be removed to produce a balanced gas. This excess $CO_2$ is removed by scrubbing with a physical absorbent (e.g., methanol, as in the Rectisol process) or a chemical absorbent (e.g., MEA, as in the amine process). These processes are typically complicated and expensive, requiring multiple columns for $CO_2$ absorption and solvent regeneration.

After $CO_2$ removal, the treated syngas is blended with the untreated CO-rich gas to give balanced syngas; this is then fed to the methanol synthesis reactor loop. There are two types of gas-phase methanol synthesis reactors which are most commonly used. Both use packed beds of catalyst and are operated with recycle. The first, known as the adiabatic, quench-type reactor (ICI low-pressure reactor), typically operates at 440 to 520 °F and 725 to 1500 psia, with a $CuO-ZnO-Al_2O_3$ catalyst. The reactor may be a single, continuous packed bed or a staged packed bed. A portion of the combined feed gas and recycle enters the front end of the reactor, and the remainder is divided into several streams which are injected at various points in the bed (or between stages) to control reaction temperature. The second type of reactor is known as the isothermal, boiling water reactor (Lurgi low-pressure reactor). It typically operates at 460 to 520 °F and 750 to 1000 psia, also using a $CuO-ZnO-Al_2O_3$ catalyst. It is similar in concept to the Lurgi shift vessel described above. In this case, however, pressurized water boils on the shell side to remove reaction heat as syngas is passed through the catalyst-filled tubes. Other methanol synthesis reactors, such as the Haldor-Topsoe and Mitsubishi reactors, are less frequently used and rely on essentially the same principles as the two described here.

There are three major drawbacks to a conventional gas-phase design for producing methanol from coal-derived syngas. First, the feed gas must be balanced before methanol synthesis, necessitating a shift section and subsequent $CO_2$ removal section. Second, the shift and methanol synthesis reactions must be accomplished in separate steps, even though synthesis and LTS use basically the same catalyst (i.e., both formulations comprise CuO, ZnO and $Al_2O_3$). Third, all gas-phase packed bed reactors have the problem of temperature control. Because the gas is a poor conductor of heat, the beds are subject to hot spots, which can cause rapid catalyst deactivation. This is true even with the so-called isothermal design. The inability to remove reaction heat limits the methanol synthesis reactor in the amount of reaction which can take place per pass through the bed. This same problem of overheating also limits the amount of reaction allowed in shift reactors.

An alternative to gas-phase methanol synthesis is the liquid phase methanol process (U.S. Pat. 3,888,896, 4,031,123, and 4,537,876), in which the $CuO-ZnO-Al_2O_3$ catalyst is in powder or small particle form and is slurried in a hydrocarbon oil. The synthesis reactions:

$CO + 2H_2 \rightarrow CH_3OH$ and $CO_2 + 3H_2\ CH_3OH + H_2O$

take place as the syngas is bubbled up through the slurry. The oil is an effective heat transfer medium, allowing the reaction heat to be removed by a heat exchanger within the reactor (Kobel-type), or in an external, circulating liquid heat-exchange loop. Because of this heat removal ability the reactor operates in a genuinely isothermal manner without hot spots; the bubbling action and slurry circulation assure that the reactor is well-mixed. Typical methanol synthesis conditions are 440 to 520 °F and 750 to 1500 psia in a liquid phase methanol reactor.

The liquid phase methanol process offers two advantages over the conventional processes for all-methanol production from coal-derived syngas. First, the reactor can process gas with a high CO content, without accelerated catalyst deactivation. As with conventional processes, methanol production can be maximized by using a balanced gas; however, unlike the conventional processes, the liquid phase methanol process has the added flexibility to accept CO-rich gas as well. What makes this possible is not clear. If the reason for deactivation in conventional reactors is excessive catalyst temperatures, then the excellent heat transfer between the liquid phase and the catalyst particles may be the reason. Second, the ability to efficiently remove reaction heat means that the methanol conversion per pass is not constrained by increasing catalyst temperature.

Yet another process for producing methanol from coal-derived syngas is the ICI "Dual Cycle" synthesis scheme (European Patent Application No. 81303777.7). This process can handle a moderately CO-rich feed gas (liquid phase methanol can handle gases which are more CO-rich) by integration of shift, $CO_2$ removal, and methanol synthesis steps. Examples imply that the dual cycle process is limited because the CO partial pressure is kept low (no higher than about 230 psia), which is inherently disfavorable to methanol formation. Also, the process runs on less than the stoichiometric amount of hydrogen, necessitating the rejection of waste CO from the process. The overall conversion of CO to methanol is roughly 85-90%, compared to the 96+% normally achieved and required in a conventional, coal-based all-methanol plant.

Finally, in the Tarhan process (U.S. Pat. # 3,689,575), shift and methanol synthesis take place simultaneously using a catalyst suspended in liquid water at 356 to 716°F. The major drawback to this process is the very high pressure (over 3600 psia) required to keep the water from boiling away.

These latter three processes have not yet achieved commercial importance.

## SUMMARY OF THE INVENTION

The present invention is an improvement to a process for the conversion of a carbon monoxide-rich synthesis gas to a crude methanol product, wherein the carbon monoxide-rich synthesis gas is balanced by reacting the carbon monoxide-rich synthesis gas with water in the presence of a water/gas shift catalyst thereby decreasing carbon monoxide content and increasing hydrogen and carbon dioxide content. Wherein at least a portion of the carbon dioxide content is removed from the reacted (shifted) synthesis gas, thereby producing a balanced synthesis gas. Further, wherein the process, the balanced synthesis gas is then reacted in the presence of a methanol synthesis catalyst to produce a crude methanol product.

The improvement to the process for balancing the carbon monoxide-rich synthesis gas while simultaneously producing quantities of crude methanol product comprises combining the water/gas shift and methanol synthesis reactions in a single step by reacting the carbon monoxide-rich synthesis gas with water in the presence of a catalyst in a liquid phase reactor whereby both a quantity of crude methanol product and a reduced carbon monoxide content and increased hydrogen and carbon dioxide content synthesis gas suitable for carbon dioxide removal and further conversion to methanol are produced. The water added to the liquid phase reactor can be beneficially introduced as a liquid. The catalyst in the liquid phase reactor can be any appropriate methanol synthesis catalyst or a mixture of a methanol synthesis catalyst and a low temperature shift catalyst. The catalyst concentration in the liquid phase methanol reactor can be in the range from about 5 to about 50 weight percent. The improvement to the process of the present invention is particularly suited to CO-rich synthesis gases having an R value less than 2.0.

The reduced carbon monoxide content and increased hydrogen and carbon dioxide content synthesis gas produced in the liquid phase reactor can be further processed. In this further processing, at least a portion of the carbon dioxide content of the reduced carbon monoxide content and increased hydrogen and carbon dioxide content synthesis gas is removed and the resultant synthesis gas is further reacted in the presence of a methanol synthesis catalyst to produce crude methanol product.

## BRIEF DESCRIPTION OF THE DRAWING

Figure 1 is a plot showing the effect of water, expressed as molar $H_2O/CO$ ratio entering the liquid phase methanol reactor, on methanol productivity and on the hydrogen content leaving the liquid phase methanol reactor.

Figure 2 is a schematic diagram of an embodiment of the methanol synthesis section of an all-methanol plant according to the present invention.

Figure 3 is a plot showing the effect that recycle ratio (recycle flow/fresh feed) has on the carbon conversion to methanol using synthesis gas.

Figure 4 is a block diagram of a conventional coal conversion process to methanol using gas phase reaction technology.

Figure 5 is a block diagram of an embodiment of the present invention for a coal conversion process to methanol using liquid phase reaction technology.

## DETAILED DESCRIPTION OF THE INVENTION

The present invention is an improvement to a methanol production process with a CO-rich synthesis gas feed. The improvement to the process is the combination of the methanol synthesis and water-gas shift reactions in order to simultaneously produce quantities of crude methanol product and a balanced syngas. The improvement of the present invention replaces the use of a single shift step in order to balance the synthesis gas. As mentioned earlier, a balanced synthesis gas is a synthesis gas with the proper stoichiometric amounts of carbon monoxide, hydrogen and carbon dioxide to react all of the components to produce methanol. The present invention is based on the fact that if water is added to the CO-rich syngas feed to a liquid phase methanol reactor, the water-gas shift and methanol synthesis reactions will take place simultaneously.

In fact, if no water is added the reverse water-gas shift reaction is known to take place in either liquid or gas-phase reactors. The addition of a significant amount of water simply forces the equilibrium in the forward direction (i.e., $CO + H_2O \rightarrow H_2 + CO_2$).

Several advantages of the liquid phase methanol reactor have already been mentioned. An additional advantage is seen when considering water addition. In contrast to conventional technologies, liquid water can be added directly to the liquid-phase reactor. This saves the cost of generating high-pressure process steam, and also reduces the net heat which must be removed from the reactor. A conventional gas-phase reactor cannot accept a liquid water feed because thermal shock and rapid vaporization can break up and destroy the catalyst tablets. In addition, water vapor which is added must be kept well above its dew point to prevent condensation and subsequent quenching of the bed due to its plug flow operation.

Although the addition of steam to a liquid phase methanol reactor was considered in EPRI Report AF-1291 (December 1979, p. 5-3), wherein the concept is discussed, and laboratory data is presented for two syngas compositions, the data indicated that methanol productivity decreases as water is added. It was reported that water addition always reduces methanol productivity, especially for gases that already have the required $H_2/CO$ stoichiometry, and that for non-stoichiometric synthesis gases, the fall off in productivity with increasing steam/CO ratio is slower.

The experimentation behind the present invention, on the other hand, shows results which are surprising relative to those in the EPRI report. Figure 1 shows the effect of water, expressed as the molar $H_2O/CO$ ratio entering the liquid phase methanol reactor, on methanol productivity (mmolMeOH/hr-gm catalyst) and on the molar $H_2/CO$ ratio (a measurement of the extent of the water-gas shift reaction) leaving the liquid phase methanol reactor. This graph illustrates two important points. First, the methanol productivity curve goes through a maximum, showing that water indeed can be used to boost methanol productivity. This maximum was not seen or even suspected in the data reported in EPRI Report AF-l29l. Second, adding water increases the hydrogen content in the effluent. Although the $CO_2$ produced from the shift reaction prevents a stoichiometrically balanced effluent, the proper amount of $CO_2$ can be removed later to give a balanced gas, if desired. Thus, adding a precise amount of water results in increased methanol production relative to dry CO-rich gas feed as well as a substantial production of $H_2$ via the shift reaction. Adding more water results in increased $H_2$ production at some sacrifice to methanol productivity.

The proposed methanol synthesis section of an all-methanol plant is shown in Figure 2. With reference to Figure 2, CO-rich synthesis gas and water (liquid or vapor) are fed to the process via lines 1 and 3, respectively, combined into a combined feedstream, and fed to liquid phase reactor 7 via line 5, wherein the synthesis gas and water react in the presence of a catalyst. Alternatively, liquid water or steam can be added directly to reactor 7 without first being combined with the synthesis gas. Liquid phase methanol reactor 7 can be operated in either a slurry or ebullated mode. In the case of the slurry mode, a powdered methanol synthesis catalyst of the type described above (e.g., $CuO/ZnO/Al_2O_3$) is slurried in a liquid medium (e.g light paraffinic or cycloparaffinic oils). Alternatively, a mixture of powdered methanol synthesis catalyst and low temperature shift catalyst be used in reactor 7. The concentration of catalyst can range from about 5 to 50 wt%. In the case of an ebullated mode, a granulated catalyst is fluidized in a liquid medium. Liquid phase reactor 7, like all other liquid phase reactors in the process, operates within the conventional understanding of a liquid phase reactor and in a similar manner as herein described.

The effluent removed via line 9 from liquid phase reactor 7 is cooled and subsequently separated in separator 11 into a liquid and vapor stream. The primary purpose of separator 11 is to recover and recycle the liquid medium which was vaporized and entrained in the reactor effluent. The liquid stream is recycled via line 13 to liquid phase reactor 7. The vapor stream, in line 15, is further cooled and fed to $CO_2$ absorber 17. In $CO_2$ absorber 17, the synthesis gas flows upward and is contacted stage-wise with downward flowing cold methanol solvent (i.e. a Rectisol wash column). $CO_2$, water, and methanol in the synthesis gas are preferentially absorbed in the wash solvent. The absorbed $CO_2$ in bottoms stream 21 is removed via stream 27 from methanol regeneration unit 23. The absorbed methanol and water in bottoms stream 21 are removed via stream 29 as crude methanol product. Regeneration is achieved in the conventional manner (i.e., via pressure reduction flashing and/or steam regeneration). Reactivated methanol solvent stream 25 is cooled and pumped to $CO_2$ absorber 11 to complete the absorber/regenerator cycle. $CO_2$-lean and essentially water- and methanol-free synthesis gas is removed via line 19 from the overhead section of $CO_2$ absorber 17 and fed to liquid phase reactor 31 for further conversion of the synthesis gas to methanol. Reactor 31 contains only methanol synthesis catalyst, at a concentration of about 5-50 wt%.

The vapor effluent from liquid phase reactor 31 is removed via line 33, cooled and separated into liquid and vapor streams in separator 35. As with separator 11, the primary purpose of separator 35 is to recover and recycle the liquid medium which was vaporized and entrained in the reactor effluent. The liquid stream from separator 35 is recycled to liquid phase reactor 31 via line 37. The vapor stream from separator 35, in line 39, is further cooled in heat exchanger 41 so as to condense out the methanol in the stream. This cooled, condensed methanol/vapor stream is then fed to separator 43 for recovery of the condensed methanol. The liquid bottoms of separator 43 are removed via line 45 and recovered as crude methanol product. The overhead of separator 43, which comprises primarily unconverted synthesis gas is compressed and conveyed via line 47 combined with recycle synthesis gas (line 69) forming line 49, which is fed to liquid phase reactor 51 for conversion of the synthesis gas to methanol.

The vapor effluent from liquid phase reactor 51 is removed via line 53, cooled and separated in separator 55. As with separators 11 and 35, the primary purpose of separator 55 is to recover and recycle the liquid medium which was vaporized and entrained in the rector effluent. The liquid stream from separator 55 is recycled to liquid phase reactor 51 via line 57. The vapor stream from separator 55, in line 59, is further cooled and then separated in separator 61. The overhead of separator 61, in line 63, after the removal of purge stream 67, is compressed and united with the synthesis gas, in line 47, to form liquid phase reactor 51 feedstream 49. The liquid bottoms of separator 61, in line 65, are removed as crude methanol product.

The crude methanol product streams in lines 29, 45 and 65 can be united into a single product stream and processed further for increased product purity.

This scheme, as depicted in Figure 2, was developed to give the same high carbon conversion to methanol, 96%, as the conventional gas-phase methanol plant described in EPRI Report AP-I962 (August, 1981). The design is based on desulfurized CO-rich gas feed from a Texaco coal gasifier, which has the following nominal composition:

| | |
|---|---|
| CO | 51% |
| $CO_2$ | 13 |
| $H_2$ | 35 |
| $N_2$ | 1 |
| | 100 |

Conversions within each reactor were based on approaches to equilibrium which were derived from the appropriate laboratory and pilot plant data.

Although Figure 2 addresses the production of only methanol from synthesis gas, there exists some variations for coproducing other chemicals such as dimethyl ether, higher alcohols, hydrogen, synthetic natural gas, and even liquid hydrocarbons using Fischer-Tropsch synthesis. By adjusting the amount of water added to reactor 7 and the amount of $CO_2$ removed in absorber 17, it is possible to produce a wide range of synthesis gas compositions. The possible synthesis gas compositions would include $CO:H_2$ ratios greater than the orginal feed through those which are predominantly hydrogen. Reactors 31 and 51 can be operated at alternate temperatures and pressures using other catalysts thus producing all of the above mentioned chemicals.

In Figure 2, stream 1 represents the desulfurized, CO-rich gas from a Texaco gasifier. Stream 3 is liquid water at a flowrate such that the combined streams (line 5) have a molar ratio of $H_2O/CO$ = 0.58. Although liquid water is described for use in the above process scheme, steam can also be used. With methanol synthesis catalyst alone in reactor 11, the $H_2O/CO$ ratio of 0.58 causes sufficient hydrogen to be produced so that after $CO_2$ removal in $CO_2$ absorber 17, the feed to second liquid phase reactor 31 is balanced. Since methanol productivity is significantly decreased in this range of water content, using a $H_2O/CO$ ratio of 0.58 may not be the optimum level from an economic standpoint. Stream 5 is fed to liquid phase reactor 7, which operates at about 482°F and 895 psia. Reaction heat is removed in an external heat exchange loop which produces 310 psia saturated steam. Roughly 10% of the feed CO is converted to methanol, and 49% is converted to $CO_2$ and $H_2$; 84% of the water is consumed. The reactor effluent, line 9, is cooled by producing steam and/or preheating boiler feedwater, then by exchange against the liquid phase reactor 31 feed in line 19, and finally by refrigeration so that it enters $CO_2$ absorber 17 at 25° F. $CO_2$ is scrubbed and product methanol recovered from the gas by contact with liquid methanol at -60° F. At this temperature almost all of the methanol vapor and residual water vapor condense. The effluent gas from the absorber contains 3% $CO_2$, and is balanced. It is heated to 90° F against the liquid phase reactor 7 effluent in line 15, and then fed to liquid phase reactor 31.

The second reactor 31 can be either a liquid phase methanol reactor or a conventional gas-phase methanol synthesis reactor, since the feed is balanced. It has been assumed here that it is a liquid phase methanol reactor operating at 482° F and 845 psia. As in liquid phase reactor 7, reaction heat is removed by raising 310 psia saturated steam in an external heat-exchange loop. Approximately 50% of the feed CO from stream 19 is converted to methanol. Liquid phase reactor 31 effluent is cooled to 90° F by producing steam and/or preheating boiler feedwater followed by heat exchange with cooling water. The two-phase mixture is then separated, with most of the methanol coming out in the liquid. The vapor is then compressed to 1510 psia and sent to the liquid phase reactor 51.

The third reactor 51 can also be either a gas-phase or liquid-phase methanol synthesis reactor. A liquid-phase reactor operating at 482° F has again been assumed, with the same heat removal scheme used before. Liquid phase reactor 51 operates at a higher pressure (1500 psia) to give the methanol synthesis a greater driving force through reaction stoichiometry and higher reactant partial pressures; it is run with recycle to increase carbon conversion in this final stage. The compressed gas from liquid phase reactor 31, line 47, is combined with the recycle stream 69 and fed to liquid phase reactor 51. For the case shown here, the recycle ratio (stream 69/stream 47) is 1.7. This recycle ratio gives an overall carbon conversion to methanol which matches that of the EPRI base case. The process is by no means limited to this recycle ratio; values from 0 to 10 might well be used in other cases.

Figure 3 shows the effect of recycle ratio on overall carbon conversion for this case. The required recycle ratio for a gas-phase plant is considerably higher (typically 3 to 6) because of the single reactor and limited conversion per pass. Also, since the single gas-phase reactor is processing the entire fresh feed compared to reactor 51 in which a large amount of feed has been already been converted to methanol, the total flow in the recycle loop is much higher in the gas-phase case. These high flows result in a costly recycle loop for the conventional gas-phase process.

In Figure 2, Stream 49 contains about 10% inerts and 13% CO; about 78% of the CO is converted to methanol in liquid phase reactor 51. The reactor effluent vapor stream 53 is cooled as in liquid phase reactor 31, and separated to retrieve the crude methanol product. Taking a small purge via line 67 from stream 63 prevents inerts buildup. The unreacted gas, via line 69, is compressed and fed back to the reactor.

In order to demonstrate the efficacy and benefits of the present invention, in particular when compared to the process suggested in the EPRI AP-1962 Report, the following examples are offered.

## EXAMPLES

### Example I

Figure 4 shows a block flow diagram of an all-methanol plant as depicted in EPRI Report AP-1962, which uses conventional gas-phase methanol synthesis technology. The corresponding material balance, shown in Table I, was scaled to a 3,000 TPD coal feed basis from the EPRI report.

TABLE I

| ALL-METHANOL PLANT WITH GAS-PHASE SYNTHESIS FLOW RATES SHOWN ARE IN LBMOL/HR | | | | | | |
|---|---|---|---|---|---|---|
| | STREAM NAME & NUMBER | | | | | |
| COMPONENT | OXYGEN 2 | RAW GAS 3 | CO-RICH GAS 4 | $CO_2$ STREAM 6 | $H_2S$ STREAM 5 | FRESH FEED 7 |
| $H_2$ | 0 | 8,702 | 14,150 | 29 | 1 | 14,120 |
| CO | 0 | 11,789 | 6,342 | 97 | 1 | 6,244 |
| $CO_2$ | 0 | 4,482 | 9,946 | 8,748 | 537 | 661 |
| $N_2(CH_4\text{-}Ar)$ | 173 | 409 | 409 | 10 | 2 | 397 |
| $O_2$ | 8,459 | 0 | 0 | 0 | 0 | 0 |
| $H_2S$ | 0 | 287 | 303 | 0 | 303 | 0 |
| COS | 0 | 19 | 3 | 0 | 3 | 0 |
| $H_2O$ | 0 | 16,608 | 53 | 0 | 0 | 0 |
| $CH_3OH$ | 0 | 0 | 0 | 0 | 0 | 0 |
| TOTAL | 8,632 | 42,296 | 31,206 | 8,884 | 846 | 21,422 |
| TOTAL (#/HR) | 275,878 | 866,816 | 667,206 | 387,986 | 34,179 | 244,066 |
| | STREAM NAME & NUMBER | | | | | |
| COMPONENT | REAC INLET 8 | FLASH GAS 9 | RECYC 10 | PURGE 11 | MEOH 12 | |
| $H_2$ | 57,307 | 43,502 | 43,187 | 315 | 2 | |
| CO | 27,837 | 21,750 | 21,593 | 157 | 1 | |
| $CO_2$ | 16,856 | 16,311 | 16,195 | 116 | 29 | |
| $N_2(CH_4\text{-}Ar)$ | 53,031 | 53,031 | 52,634 | 383 | 14 | |
| $O_2$ | 0 | 0 | 0 | 0 | 0 | |
| $H_2S$ | 0 | 0 | 0 | 0 | 0 | |
| COS | 0 | 0 | 0 | 0 | 0 | |
| $H_2O$ | 540 | 544 | 540 | 4 | 541 | |
| $CH_3OH$ | 675 | 680 | 675 | 5 | 6,610 | |
| TOTAL | 156,246 | 135,818 | 134,824 | 980 | 7.197 | |
| TOTAL (#/HR) | 3,257,964 | 3,036,170 | 3,013,898 | 21,852 | 222.986 | |

Example II

A computer simulation of the process depicted in Figure 2 was run, again considering syngas derived from 3.000 TPD of coal. Figure 5 and its corresponding material balance (Table II) show how a staged liquid-phase methanol process with water addition can be used to produce the same amount of methanol product as in Example I from the same amount of coal. It should be noted that the liquid phase third stage comprises two reactors in this example. The liquid phase stages are designated LP-# in Table II.

TABLE II
ALL-METHANOL PLANT WITH LIQUID-PHASE METHANOL SYNTHESIS
FLOW RATES SHOWN ARE IN LBMOL/HR

| COMPONENT | OXYGEN 2 | RAW GAS 3 | CO-RICH GAS 4 | $H_2S$ STREAM 5 | WATER 6 |
|---|---|---|---|---|---|
| | STREAM NAME & NUMBER | | | | |
| $H_2$ | 0 | 8,648 | 8,645 | 3 | 0 |
| CO | 0 | 12,600 | 12,597 | 3 | 0 |
| $CO_2$ | 0 | 4,482 | 3,211 | 1,271 | 0 |
| $N_2$ ($CH_4$-Ar) | 173 | 409 | 247 | 162 | 0 |
| $O_2$ | 8,459 | 0 | 0 | 0 | 0 |
| $H_2S$ | 0 | 287 | 0 | 287 | 0 |
| COS | 0 | 19 | 0 | 19 | 0 |
| $H_2O$ | 0 | 0 | 0 | 0 | 7,289 |
| $CH_3OH$ | 0 | 0 | 0 | 0 | 0 |
| TOTAL | 8,632 | 26,445 | 24,700 | 1,745 | 7,289 |
| TOTAL (#/HR) | 275,878 | 590,472 | 518,700 | 71,772 | 131,202 |

| COMPONENT | LP-01 INLET 7 | LP-01 OUTLET 8 | LP-02 INLET 9 | $CO_2$ VENT 10 | LP-02 FLASH GAS 11 | LP-03 INLET 12 |
|---|---|---|---|---|---|---|
| | STREAM NAME & NUMBER | | | | | |
| $H_2$ | 8,645 | 12,156 | 12,067 | 0 | 7,047 | 20,021 |
| CO | 12,597 | 5,147 | 4,961 | 0 | 2,477 | 3,502 |
| $CO_2$ | 3,211 | 9,348 | 534 | 2,241 | 488 | 1,402 |
| $N_2$ ($CH_4$-Ar) | 247 | 247 | 245 | 0 | 243 | 2,801 |
| $O_2$ | 0 | 0 | 0 | 0 | 0 | 0 |
| $H_2S$ | 0 | 0 | 0 | 0 | 0 | 0 |
| COS | 0 | 0 | 0 | 0 | 0 | 0 |
| $H_2O$ | 7,289 | 1,152 | 0 | 0 | 0 | 2 |
| $CH_3OH$ | 0 | 1,313 | 0 | 0 | 53 | 104 |
| TOTAL | 31,989 | 29,363 | 17,807 | 2,241 | 10,308 | 27,832 |
| TOTAL (#/HR) | 649,902 | 649,902 | 193,888 | 98,604 | 113,908 | 287,180 |

| | STREAM NAME & NUMBER | | | | | |
|---|---|---|---|---|---|---|
| | LP-03 FLASH GAS 13 | LP-03 RECYC 14 | LP-03 PURGE 15 | LP-01 CRUDE 16 | LP-02 CRUDE 17 | LP-03 CRUDE 18 |
| $H_2$ | 14,157 | 12,974 | 1,183 | 89 | 15 | 27 |
| CO | 1,119 | 1,025 | 94 | 185 | 11 | 4 |
| $CO_2$ | 994 | 914 | 80 | 6,573 | 27 | 48 |
| $N_2$ ($CH_4$-Ar) | 2,788 | 2,558 | 230 | 2 | 2 | 13 |
| $O_2$ | 0 | 0 | 0 | 0 | 0 | 0 |
| $H_2S$ | 0 | 0 | 0 | 0 | 0 | 0 |
| COS | 0 | 0 | 0 | 0 | 0 | 0 |
| $H_2O$ | 2 | 2 | 0 | 1,152 | 19 | 360 |
| $CH_3OH$ | 56 | 51 | 5 | 1,313 | 2,440 | 2,787 |
| TOTAL | 19,116 | 17,524 | 1,592 | 9,314 | 2,514 | 3,240 |
| TOTAL (#/HR) | 188,850 | 173,272 | 15,578 | 357,382 | 80,008 | 98,354 |

A comparison of the two examples is shown in Table III. It is seen that for this scale plant, the process of the present invention requires two less reactors, and has a significantly lower recycle flow.

TABLE III

| COMPARISON OF GAS-PHASE ALL-METHANOL PLANT TO COMBINED SHIFT/SYNTHESIS PLANT | | |
|---|---|---|
| | CONVENTIONAL GAS-PHASE | LIQUID-PHASE SHIFT/SYNTHESIS |
| COAL FEED: TPD | 3,000 | 3,000 |
| TOTAL METHANOL (PURE) PRODUCED: TPD | 2,500 | 2,500 |
| SHIFT REACTORS | 2 | 0 |
| METHANOL SYNTHESIS REACTORS | 4 | 4 |
| RECYCLE FLOW: LB-MOL HR | 135,000 | 17,500 |
| CATALYST: LB | | |
| SHIFT | 158,000 | |
| METHANOL SYNTHESIS | 530,000 | 522,000 |
| TOTAL | 688,000 | 522,000 |

The present invention has been described with reference to a specific embodiment thereof. This embodiment should not be considered a limitation on the scope of the present invention; the scope of which should be ascertained by the following claims.

## Claims

1. In a process for the conversion of a carbon monoxide-rich synthesis gas to a crude methanol product, wherein the carbon monoxide-rich synthesis gas is balanced by reacting the carbon monoxide-rich synthesis gas with water in the presence of a water/gas shift catalyst via a water/gas shift reaction thereby decreasing carbon monoxide content and increasing hydrogen and carbon dioxide content of the synthesis gas; wherein at least a portion of the carbon dioxide content is removed from the reacted (shifted) synthesis gas, thereby producing a balanced synthesis gas; and wherein the balanced synthesis gas is then reacted in the presence of a methanol synthesis catalyst to produce a crude methanol product, the improvement for balancing the carbon monoxide-rich synthesis gas while simultaneously producing quantities of crude methanol product comprises combining the water/gas shift and methanol synthesis reactions into a single step by reacting the carbon monoxide-rich synthesis gas with water in the presence of a catalyst in a liquid phase reactor whereby both a quantity of crude methanol product and a reduced carbon monoxide content and increased hydrogen and carbon dioxide content synthesis gas suitable for carbon dioxide removal and further conversion to methanol are produced.

2. The process of Claim 1 wherein the CO-rich synthesis gas has an "R" value of less than 2.0.

3. The process of Claim 1 wherein the water reacted with the carbon monoxide-rich synthesis gas in the liquid phase reactor is introduced to the reactor as liquid water.

4. The process of Claim 1 wherein the catalyst in the liquid phase reactor comprises a methanol synthesis catalyst.

5. The process of Claim 1 wherein the catalyst in the liquid phase reactor comprises a mixture of a methanol synthesis catalyst and a low temperature shift catalyst.

6. The process of Claim 1 wherein concentration of the catalyst in the liquid phase reactor is in the range from 5 to 50 weight percent.

7. The process of Claim 1 which further comprises removing at least a portion of the carbon dioxide content of the reduced carbon monoxide content and increased hydrogen and carbon dioxide content synthesis gas and reacting the resultant synthesis gas in the presence of a methanol synthesis catalyst to further produce crude methanol product.

8. The process of Claim 7 wherein the reacting of the resultant synthesis gas is carried out in a liquid phase methanol reactor system comprising at least one liquid phase reactor.

FIG. 1

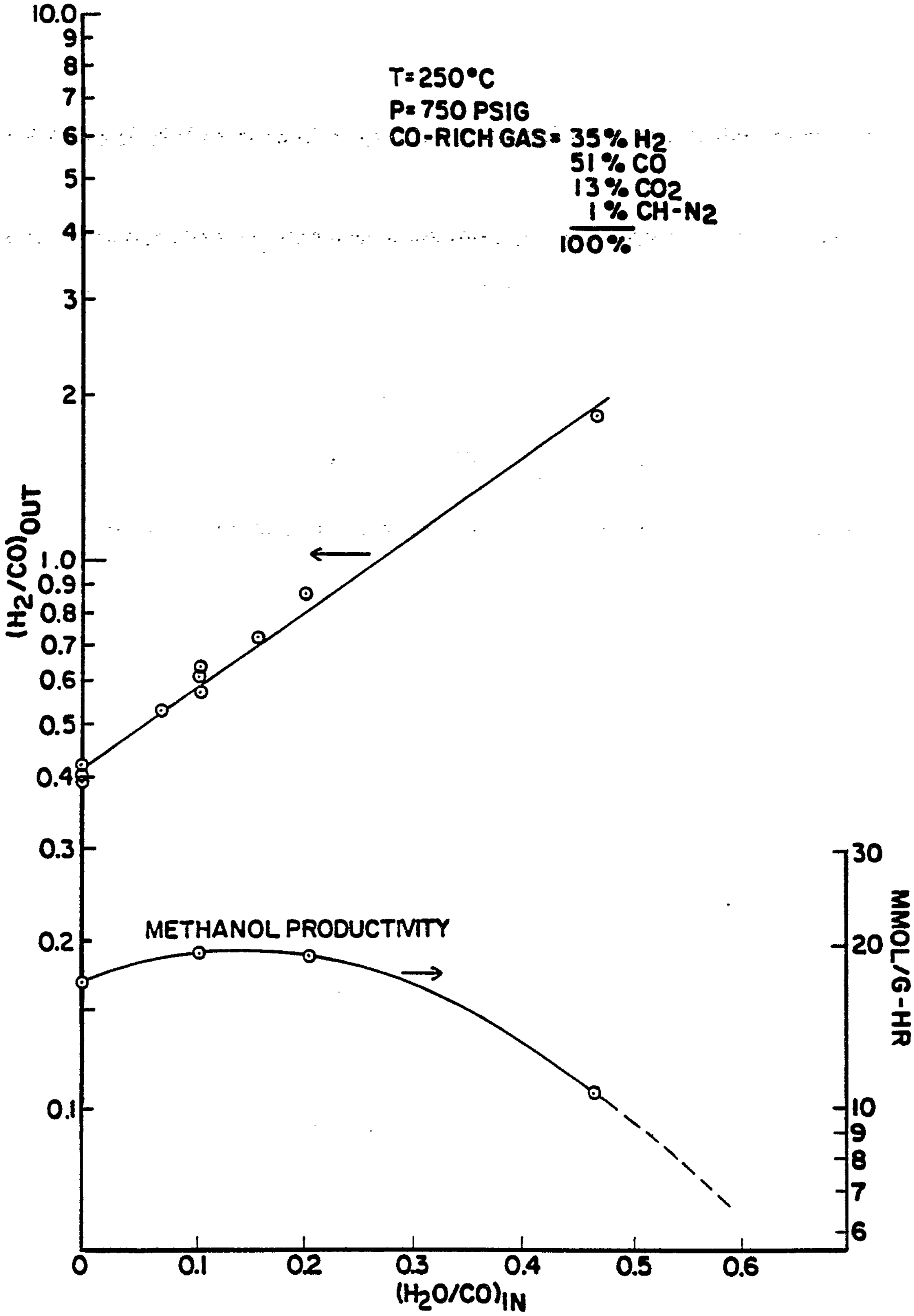

T=250°C
P=750 PSIG
CO-RICH GAS= 35% H2
51% CO
13% CO2
1% CH-N2
100%
METHANOL PRODUCTIVITY
(H2/CO)OUT
MMOL/G-HR
(H2O/CO)IN
10.0
9
8
7
6
5
4
3
2
1.0
0.9
0.8
0.7
0.6
0.5
0.4
0.3
0.2
0.1
0
0.1
0.2
0.3
0.4
0.5
0.6
30
20
10
9
8
7
6

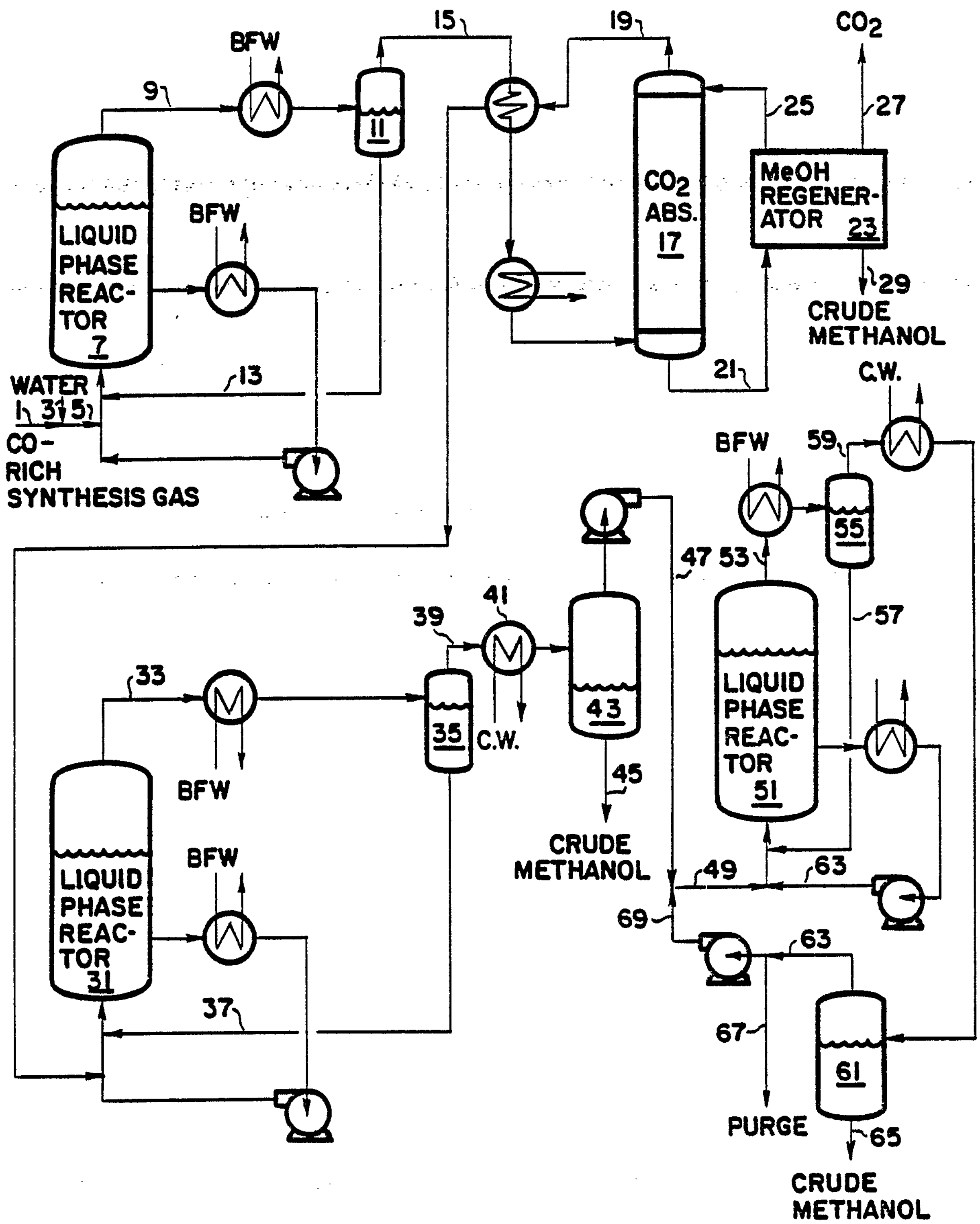
BFW
9
11
15
19
CO2
25
27
CO2 ABS. 17
MeOH REGENERATOR 23
29
CRUDE METHANOL
LIQUID PHASE REACTOR 7
BFW
21
13
WATER
1 3 5
CO-RICH SYNTHESIS GAS
C.W.
BFW
59
55
47 53
57
41
39
33
43
35
C.W.
BFW
LIQUID PHASE REACTOR 51
45
CRUDE METHANOL
49
63
LIQUID PHASE REACTOR 31
BFW
37
69
63
67
61
PURGE
65
CRUDE METHANOL

FIG. 2

FIG. 3

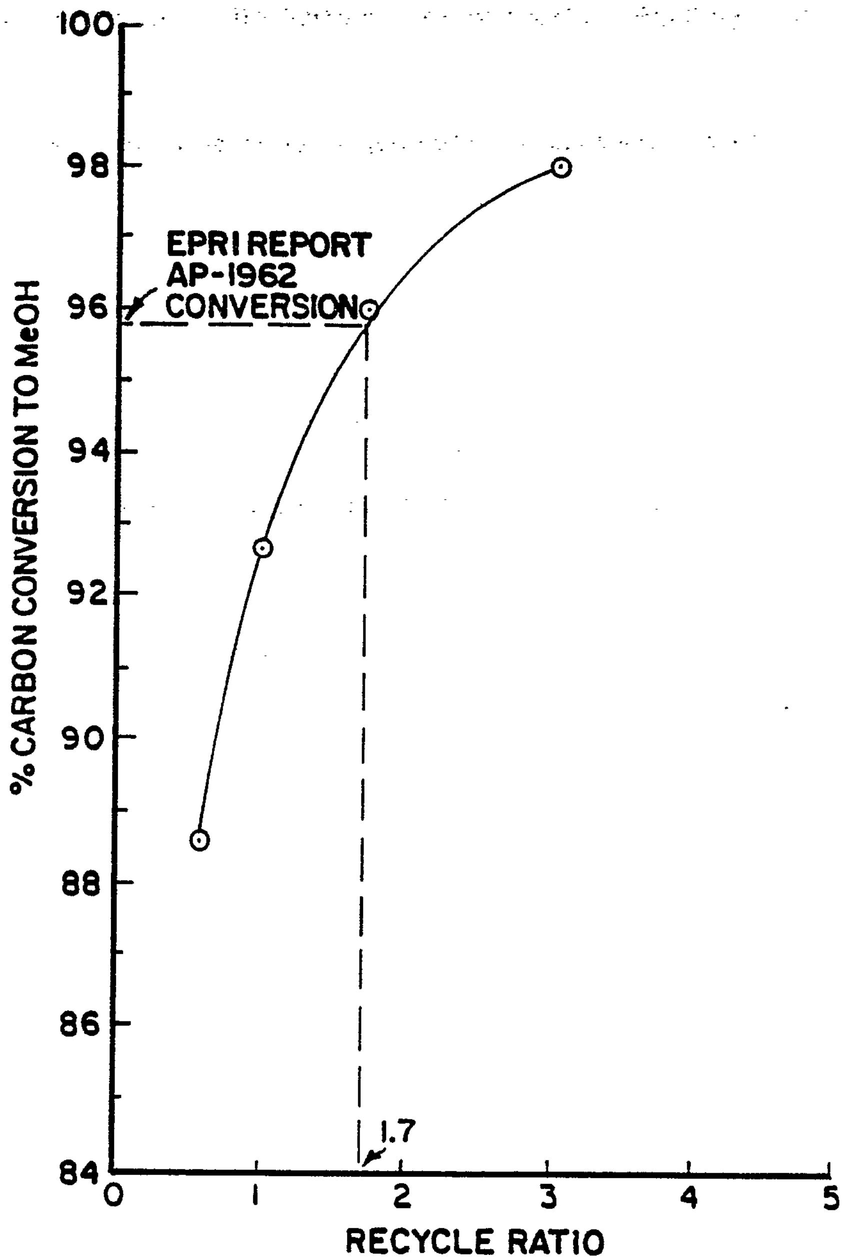
100
98
96
94
92
90
88
86
84
EPRI REPORT
AP-1962
CONVERSION
% CARBON CONVERSION TO MeOH
1.7
0
1
2
3
4
5
RECYCLE RATIO

FIG. 4

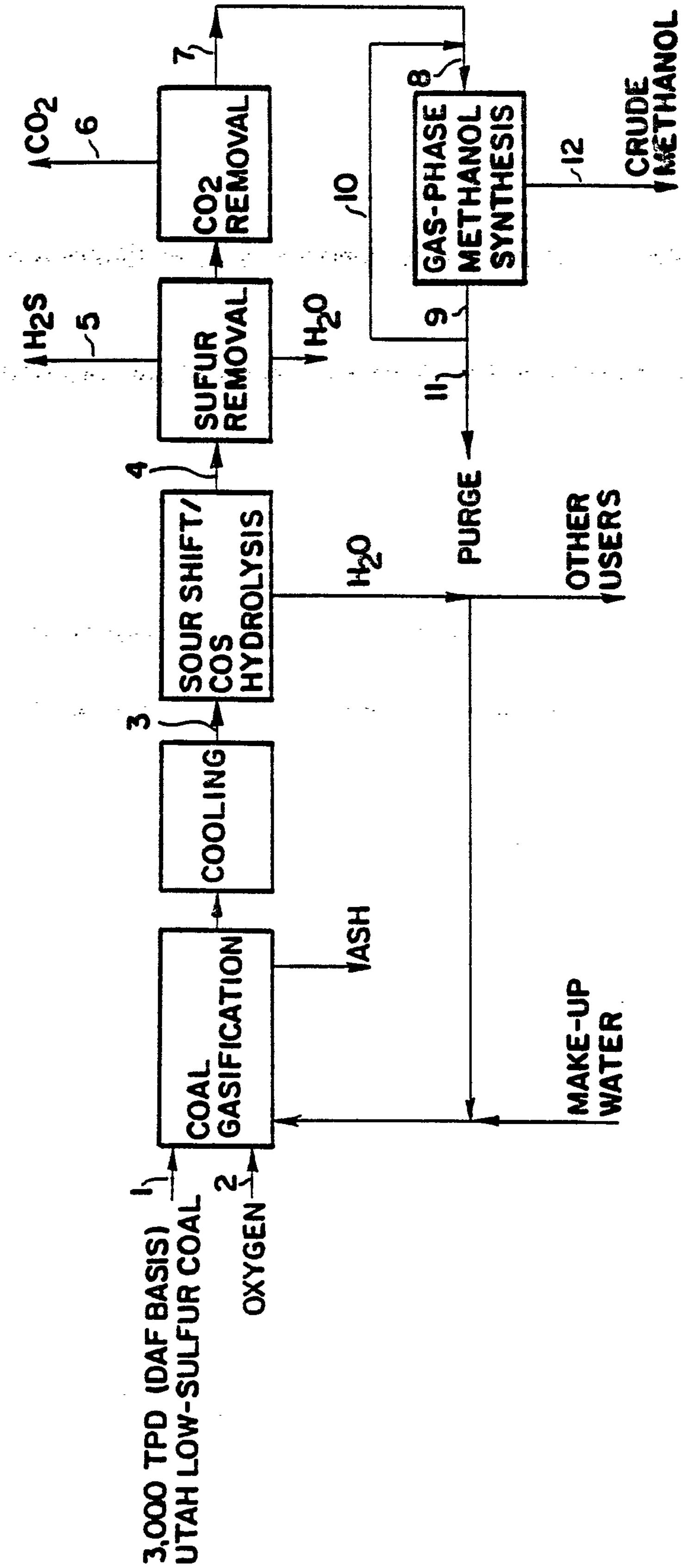
3,000 TPD (DAF BASIS) UTAH LOW-SULFUR COAL
1
OXYGEN
2
COAL GASIFICATION
ASH
COOLING
3
SOUR SHIFT/ COS HYDROLYSIS
$H_2O$
4
SUFUR REMOVAL
$H_2S$
5
$H_2O$
$CO_2$ REMOVAL
$CO_2$
6
7
8
GAS-PHASE METHANOL SYNTHESIS
9
10
11
12
CRUDE METHANOL
PURGE
OTHER USERS
MAKE-UP WATER

FIG. 5

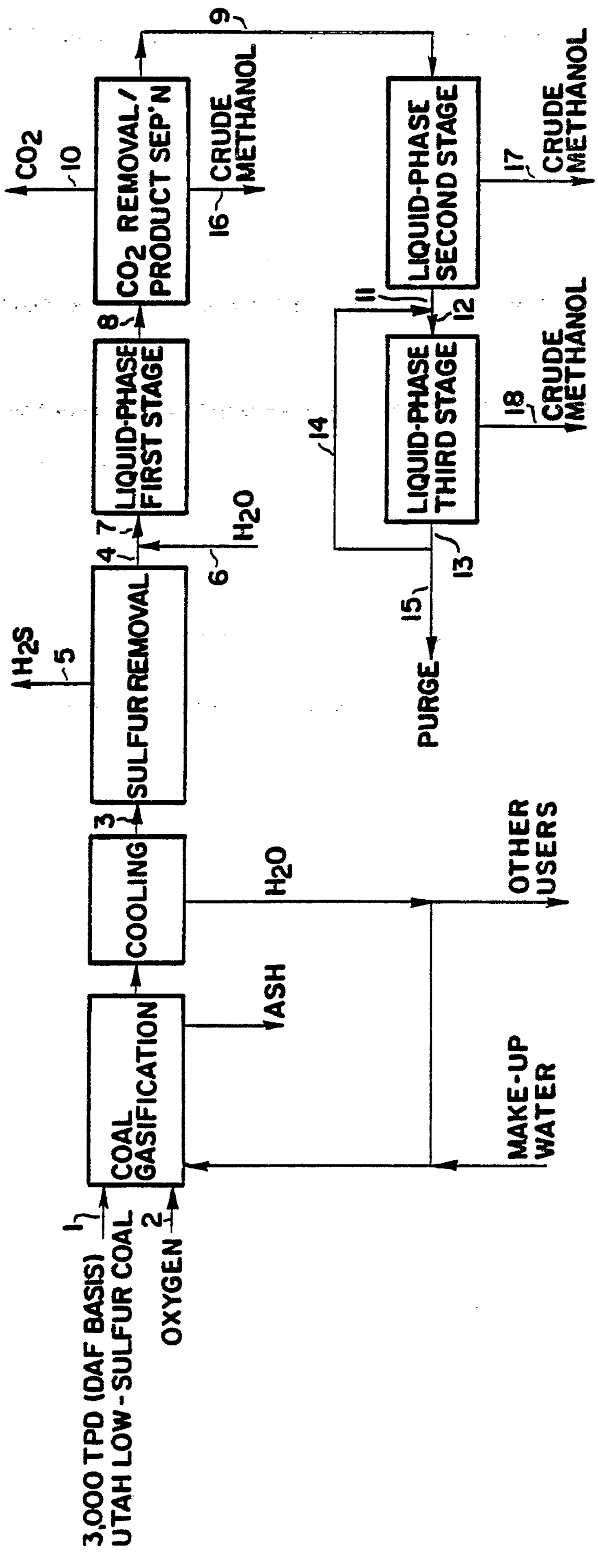
3,000 TPD (DAF BASIS) UTAH LOW-SULFUR COAL
1
OXYGEN
2
COAL GASIFICATION
ASH
COOLING
3
H2O
OTHER USERS
MAKE-UP WATER
SULFUR REMOVAL
H2S
5
4
7
6
H2O
LIQUID-PHASE FIRST STAGE
8
CO2 REMOVAL / PRODUCT SEP'N
CO2
10
16
CRUDE METHANOL
9
LIQUID-PHASE SECOND STAGE
17
CRUDE METHANOL
11
12
LIQUID-PHASE THIRD STAGE
18
CRUDE METHANOL
14
13
15
PURGE